# DEMANDE DE BREVET EUROPEEN

(11) **EP 1 046 393 A1**
(43) Date de publication de la demande: **25.10.2000**
(21) Numéro de dépôt: 00400986.6
(22) Date de dépôt: 07.04.2000
(51) Int. Cl.: A61K 7/48, A61K 7/06, C07C 319/26, C07C 51/50, C07C 315/06, C07C 209/90, C07D 333/38, C07C 45/86, C07D 213/80

(54) **Composition renfermant au moins un composé bicyclique aromatique et au moins un 2-alkyl alcan-1-ol ou un ester, et ses utilisations**

(30) Priorité: 19.04.1999 FR 9904889
(71) Demandeur: L'OREAL, 75008 Paris (FR)
(72) Inventeur: Guiramand, Carole, 91310 Linas (FR); Baldo, Francine, 92330 Sceaux (FR)
(74) Mandataire: Renard, Emmanuelle

(57) **Abrégé**

La présente invention concerne une composition renfermant au moins un composé bicyclique aromatique de formule générale (I): 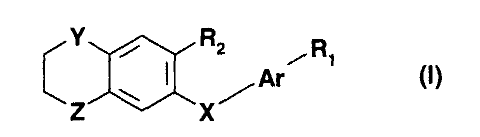 ou un sel ou analogue chiral d'un tel composé, caractérisée en ce qu'elle comprend en outre au moins un 2-alkyl alcan-1-ol comprenant de 12 à 36 atomes de carbone, ou un ester d'un tel alcool.

Le composé de formule (I) est de préférence l'acide 4-(3,5,5,8,8-pentaméthyl-5,6,7,8-tétrahydro-2-naphtylthio)benzoïque.

L'invention concerne également les utilisations de cette composition, ainsi qu'un procédé de solubilisation du composé de formule (I) ci-dessus au moyen d'au moins un 2-alkyl alcan-1-ol.

## Description

La présente invention se rapporte à une composition renfermant au moins un composé bicyclique aromatique et au moins un 2-alkyl alcan-1-ol comprenant de 12 à 36 atomes de carbone, ou un ester d'un tel alcool, à des utilisations de ladite composition et à un procédé de solubilisation dudit composé au moyen d'au moins un 2-alkyl alcan-1-ol comprenant de 12 à 36 atomes de carbone, éventuellement alcoxylé, ou un ester d'un tel alcool.

On connaît du brevet EP 679 630 des composés bicycliques aromatiques qui sont notamment utiles dans la prévention ou le traitement de différents troubles de kératinisation, et également dans la réduction des taches pigmentaires cutanées. Ces composés constituent donc des actifs cosmétiques de choix, en particulier dans la réparation ou la lutte contre le vieillissement chronologique ou actinique de la peau, par exemple comme anti-rides.

On a toutefois constaté que ces composés étaient difficiles à solubiliser dans des compositions cosmétiques. Ces actifs ont ainsi tendance à recristalliser ou à se dégrader. Il s'ensuit une perte d'efficacité plus ou moins importante de ces compositions, selon le degré de recristallisation et/ou de dégradation, ce qui va à l'encontre de l'objectif recherché. En outre, cette recristallisation ou dégradation peut modifier la stabilité globale de ces compositions ainsi que leur aspect, ce qui peut détourner l'utilisateur de celles-ci.

Il subsiste donc le besoin de solubiliser ces composés dans un solubilisant physiologiquement acceptable.

La Demanderesse a maintenant constaté, de façon étonnante, que ces composés bicycliques aromatiques pouvaient être facilement solubilisés dans les alcools de Guerbet et leurs esters.

Les alcools dits de Guerbet sont des 2-alkyl alcan-1-ols répondant à la formule générale (II) suivante : 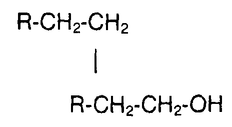 dans laquelle R désigne le même groupement aliphatique saturé.

Ces alcools sont généralement obtenus par condensation, à haute température et en présence de catalyseurs alcalins, de deux molécules d'aldéhyde pour former un aldol, puis par élimination d'eau. En raison de leur structure ramifiée, ces composés sont liquides à des températures extrêmement basses. En outre, du fait de leur poids moléculaire élevé, ils sont peu irritants, peu volatils et ont de bonnes propriétés lubrifiantes. Enfin, leur chaîne aliphatique saturée leur confère une très bonne stabilité vis-à-vis de l'oxydation à haute température.

Ces propriétés avantageuses expliquent que ces composés soient utilisés à ce jour dans de très nombreuses applications, et notamment comme émollients et agents de conditionnement dans des compositions cosmétiques très diverses.

Certains d'entre eux ont également été décrits comme solubilisants d'actifs tels que l'acide n-octanoyl-5-salicylique, l'acide caféique, l'acide kojique (EP 0 679 388) et le rétinol (EP 0 646 371 et EP 0 666 076).

Toutefois, à la connaissance de la Demanderesse, il n'a jamais été proposé d'utiliser les alcools de Guerbet pour solubiliser un composé bicyclique aromatique tel que décrit ci-après.

La présente invention a donc pour objet une composition renfermant au moins un composé bicyclique aromatique, caractérisée en ce qu'elle comprend en outre au moins un 2-alkyl alcan-1-ol comprenant de 12 à 36 atomes de carbone, ou un ester d'un tel alcool.

Elle a également pour objet un procédé de solubilisation d'un composé bicyclique aromatique, comprenant l'étape consistant à mélanger ledit composé bicylique à au moins un 2-alkyl alcan-1-ol comprenant de 12 à 36 atomes de carbone, ou à un ester d'un tel alcool. Le mélange peut être effectué à froid, à température ambiante ou à chaud, par exemple à 75°C, généralement sous agitation.

Les composés bicycliques aromatiques selon l'invention ont précisément la formule générale (I) suivante : 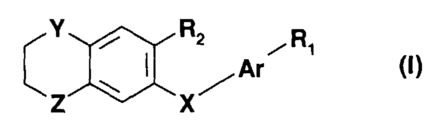 dans laquelle :
* R₁ représente un atome d'hydrogène, un radical -CH₃, un radical -CH₂-O-R₃, un radical -CH₂-O-CO-R₄, un radical -O-R₅, un radical -O-(CH₂)ₘ-(CO)ₙ-R₆, un radical -CO-R₇, un radical -CO-O-R₈, ou encore un radical -S(O)ₚ-R₉, les valeurs m, n et p ainsi que les différents radicaux R₃ à R₉ ayant la signification donnée ci-après,
* R₂ représente un atome d'hydrogène ou un atome d'halogène, un radical alkyle inférieur, un radical -NO₂, un radical -O-COR₄, un radical -OR₉ ou bien encore un radical 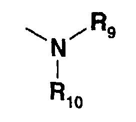 les radicaux R₄, R₉, et R₁₀ ayant la signification donnée ci-après,
* Ar représente un radical choisi parmi les radicaux de formules (a)-(e) suivantes : 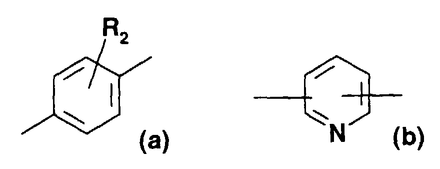 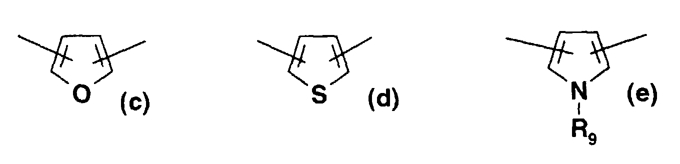 dans lesquelles R₂ a la signification ci-dessus, et R₉ celle donnée ci-après,
* X représente -O-, -S(O)ₜ- ou un radical -NR₉-, la valeur t et le radical R₉ ayant la signification donnée ci-après,
* Y et Z représentent -O-, -S(O)ₜ- ou encore un radical -CR₁₁R₁₂, la valeur t et les radicaux R₁₁ et R₁₂ ayant la signification donnée ci-après,
étant entendu que dans tout ce qui précède :
- m est un nombre entier égal à 1, 2 ou 3, n est un nombre entier égal à 0 ou 1, p est un nombre entier égal à 0, 1, 2 ou 3 et t est un nombre entier égal à 0, 1 ou 2,
- R_{3,} R_{5,} R_{9,} R_{10,} R_{11,} R₁₂ représentent, indépendamment les uns des autres, un atome d'hydrogène ou un radical alkyle inférieur,
- R₄ représente un radical alkyle inférieur,
- représente un atome d'hydrogène ou un radical alkyle inférieur,
- R₆ représente un radical alkyle inférieur ou un hétérocycle,
- R₇ représente un atome d'hydrogène, un radical alkyle inférieur ou un radical : 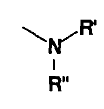 dans lequel R' et R", identiques ou différents, représentent un atome d'hydrogène, un radical alkyle inférieur, un radical mono ou polyhydroxyalkyle, un radical aryle éventuellement substitué ou un reste d'aminoacide ou de peptide ou de sucre ou encore, pris ensemble, forment un hétérocycle,
- R₈ représente un atome d'hydrogène, un radical alkyle linéaire ou ramifié ayant de 1 à 20 atomes de carbone, un radical alkényle, un radical mono ou polyhydroxyalkyle, un radical aryle ou aralkyle éventuellement substitué ou un reste de sucre ou un reste d'aminoacide ou de peptide,
- Y et Z ne peuvent représenter en même temps un atome d'oxygène ou un radical-S(O)ₜ-.

L'invention vise également les sels des composés de formule (I) ci-dessus dans les cas où le radical R₁ représente une fonction acide carboxylique ou une fonction acide sulfonique ou porte une fonction amine, ou encore lorsque le radical R₂ représente une fonction amine, ainsi que les analogues chiraux desdits composés. Lorsque les composés selon l'invention se présentent sous forme de sels, il s'agit de préférence de sels d'un métal alcalin ou alcalino-terreux, ou encore de zinc ou d'une amine organique.

Selon la présente invention, on entend par radical alkyle inférieur un radical ayant de 1 à 6 atomes de carbone, de préférence les radicaux méthyle, éthyle, isopropyle, butyle, tertiobutyle et hexyle.

Par radical alkyle linéaire ou ramifié ayant de 1 à 20 atomes de carbone, on entend notamment les radicaux méthyle, éthyle, propyle, 2-éthyl-hexyle, octyle, dodécyle, hexadécyle et octadécyle.

Par radical monohydroxyalkyle, on entend un radical ayant de préférence 2 ou 3 atomes de carbone, notamment un radical 2-hydroxyéthyle, 2-hydroxypropyle ou 3-hydroxypropyle.

Par radical polyhydroxyalkyle, on entend un radical contenant de préférence de 3 à 6 atomes de carbone et de 2 à 5 groupes hydroxyles tels que les radicaux 2,3-dihydroxypropyle, 2,3,4-trihydroxybutyle, 2,3,4,5-tétrahydroxypentyle ou le reste du pentaérythritol.

Par radical aryle, on entend de préférence un radical phényle éventuellement substitué par au moins un atome d'halogène, un hydroxyle ou une fonction nitro.

Par radical aralkyle, on entend de préférence le radical benzyle ou phénéthyle éventuellement substitué par au moins un atome d'halogène, un hydroxyle ou une fonction nitro.

Par radical alkényle, on entend un radical contenant de préférence de 2 à 5 atomes de carbone et présentant une ou plusieurs insaturations éthyléniques, tel que plus particulièrement le radical allyle.

Par reste de sucre, on entend un reste dérivant notamment de glucose, de galactose ou de mannose, ou bien encore de l'acide glucuronique.

Par reste d'aminoacide, on entend notamment un reste dérivant de la lysine, de la glycine ou de l'acide aspartique, et par reste de peptide on entend plus particulièrement un reste de dipeptide ou de tripeptide résultant de la combinaison d'acides aminés.

Par hétérocycle enfin, on entend de préférence un radical pipéridino, morpholino, pyrrolidino ou pipérazino, éventuellement substitué en position 4 par un radical alkyle en C₁-C₆ ou mono ou polyhydroxyalkyle tels que définis ci-dessus.

Lorsque le radical R₂ représente un atome d'halogène, celui-ci est de préférence un atome de fluor, de chlore ou de brome.

Selon la présente invention, les composés de formule (I) plus particulièrement préférés sont ceux pour lesquels l'une au moins, et de préférence toutes, les conditions ci-dessous sont respectées :
- R₁ est un radical -CO-R₇
- R₂ est un radical alkyl inférieur ou un radical -OR₉
- Ar représente un radical de formule (a)
- et X représente -O-, -S- ou -NR₉-.

Parmi les composés de formule (I) ci-dessus rentrant dans le cadre de la présente invention, on peut notamment citer les suivants :
- l'acide 4-(5,6,7,8-tétrahydro-5,5,8,8-tétraméthyl-2-naphthyloxy)benzoïque
- l'acide 4-(5,6,7,8-tétrahydro-5,5,8,8-tétraméthyl-2-naphthylthio)benzoïque
- l'acide 4-(5,6,7,8-tétrahydro-5,5,8,8-tétraméthyl-2 naphthylsulfinyl) benzoïque
- l'acide 4-(5,6,7,8-tétrahydro-5,5,8,8-tétraméthyl-2-naphthylsulfonyl) benzoïque
- l'acide 4-(5,6,7,8-tétrahydro-5,5,8,8-tétraméthyl-2-naphthylamino) benzoïque
- l'acide 5-(5,6,7,8-tétrahydro-5,5,8,8-tétraméthyl-2-naphthylthio)-2-thiophene carboxylique
- l'acide 4-(3,5,5,8,8-pentaméthyl-5,6,7,8-tétrahydro-2-naphthyloxy) benzoïque
- l'acide 4-(3,5,5,8,8-pentaméthyl-5,6,7,8-tétrahydro-2-naphthylthio) benzoïque
- l'acide 4-(3-éthyl-5,5,8,8-tétramethyl-5,6,7,8-tétrahydro-2-naphthyloxy) benzoïque
- l'acide 4-(3-isopropyl-5,5,8,8-tétramethyl-5,6,7,8-tétrahydro-2-naphthyloxy) benzoïque
- la 4-(5,6,7,8-tétrahydro-5,5,8,8-tétraméthyl-2-naphthyloxy) acétophénone
- le 4-(5,6,7,8-tétrahydro-5,5,8,8-tétraméthyl-2-naphthyloxy) benzaldéhyde
- l'acide 4-(3-bromo-5,6,7,8-tétrahydro-5,5,8,8-tétramethyl-2-naphtyloxy) benzoïque
- l'acide 3-méthyl-4-(5,6,7,8-tétrahydro-5,5,8,8-tétramethyl-2-naphtylthio) benzoïque
- l'acide 3-méthyl-4-(3,5,5,8,8-pentaméthyl-5,6,7,8-tétrahydro-2-naphtylthio) benzoïque
- l'acide 3-méthyl-4-(3-éthyl-5,6,7,8-tétrahydro-5,5,8,8-tétramethyl-2-naphtyloxy) benzoïque
- l'acide 6-(3,5,5,8,8-pentaméthyl-5,6,7,8-tétrahydro-2-naphtylthio) nicotinique
- l'acide 2-hydroxy-4-(3,5,5,8,8-pentaméthyl-5,6,7,8-tétrahydrol-2-naphtylthio) benzoïque
- l'acide 2-chloro-4-(3,5,5,8,8-pentaméthyl-5,6,7,8-tétrahydro-2-naphtylthio) benzoïque
- l'acide 4-(3-éthyl-5,6,7,8-tétrahydro-5,5,8,8-tétramethyl-2-naphtylthio) benzoïque
- l'acide 4-(3-isopropyl-5,6,7,8-tétrahydro-5,5,8,8-tétramethyl-2-naphtylthio) benzoïque
- l'acide 4-(3-n-propyl-5,6,7,8-tétrahydro-5,5,8,8-tétramethyl-2-naphtylthio) benzoïque
- le 4-(3,5,5,8,8-pentaméthyl-5,6,7,8-tétrahydro-2-naphtylthio) benzèneméthanol
- le 4-(3,5,5,8,8-pentaméthyl-5,6,7,8-tétrahydro-2-naphtylthio) benzaldéhyde
- la N-éthyl-4-(3,5,5,8,8-pentaméthyl-5,6,7,8-tétrahydro-2-naphtylthio) benzamide
- la N-4-hydroxyphényl-4-(3,5,5,8,8-pentaméthyl-5,6,7,8-tétrahydro-2-naphtylthio) benzamide.

On préfère en particulier utiliser, dans la composition selon l'invention, l'acide 4-(3,5,5,8,8-pentaméthyl-5,6,7,8-tétrahydro-2-naphthylthio) benzoïque.

La composition selon la présente invention renferme, comme solubilisant des composés bicycliques aromatiques ci-dessus, au moins un 2-alkyl alcan-1-ol comprenant de 12 à 36 atomes de carbone, et de préférence de 16 à 20 atomes de carbone, ou un ester d'un tel alcool.

Comme 2-alkyl alcan-1-ols convenant à une mise en oeuvre dans la présente invention, on peut citer : le butyloctanol, l'hexyldécanol, l'octyldécanol, l'alcool isostéarylique, l'octyldodécanol, le décyltétradécanol, le undécylpentadécanol, le dodécylhexadécanol, le tétradécyloctadécanol, l'hexyldécyloctadécanol, le tétradécyleicosanol et le cétylarachidol.

Tous ces composés sont disponibles dans le commerce, auprès de CONDEA VISTA sous la dénomination commerciale Isofol®, de EXXON CHEMICAL sous la dénomination Exxal® ou de JARCHEM sous la dénomination Jarcol®. On peut également utiliser le composé disponible auprès de HENKEL sous la dénomination commerciale EUTANOL G.

On peut également citer le mélange d'alcools iso-cétylique, iso-stéarylique et iso-arachidylique disponible auprès de CONDEA sous la dénomination commerciale Isofol 18T®.

En particulier, on préfère utiliser le 2-hexyl décanol.

Comme esters desdits alcools, on peut citer : l'octanoate d'octyldodécyle ; le caprylate d'hexyldécyle ; le laurate d'hexyldécyle ; le palmitate d'hexyldécyle ; le stéarate d'hexyldécyle ; et l'octyldodécyle meadowfoamate, qui est un ester d'octyldodécanol et d'acides gras dérivés d'huile de germe de *Limnanthes Alba.*

En particulier, on préfère utiliser l'octyl dodécyl meadowfoamate, qui est par exemple disponible auprès de FANNING CORPORATION sous la dénomination commerciale Meadowester gme.

La concentration en composé de formule (I) dans la composition selon l'invention est avantageusement comprise entre 0,0001% et 10% en poids, de préférence entre 0,01 et 5% en poids, plus préférentiellement entre 0,1 et 1% en poids, par rapport à l'ensemble de la composition. En outre, la quantité pondérale de 2-alkyl alcan-1-ol ou de son ester est de préférence au moins vingt fois supérieure, plus préférentiellement au moins trente fois supérieure, à la quantité pondérale de composé bicyclique aromatique. Elle représente avantageusement de 0,01% à 20%, mieux, de 1 à 10%, du poids total de la composition.
La composition selon l'invention peut se présenter sous toutes les formes galéniques normalement utilisées pour une application topique sur la peau, notamment sous forme d'une solution aqueuse, hydroalcoolique ou huileuse, d'une émulsion huile-dans-eau ou eau-dans-huile ou multiple, d'une émulsion siliconée, d'une microémulsion ou nanoémulsion, d'un gel aqueux ou huileux, d'un produit anhydre liquide, pâteux ou solide, d'une dispersion d'huile dans une phase aqueuse en présence de sphérules, ces sphérules pouvant être des nanoparticules polymériques telles que les nanosphères et les nanocapsules ou, mieux, des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse ou d'un gel. Elle peut éventuellement être appliquée sur la peau sous forme d'aérosol. Elle peut également se présenter sous forme solide, et par exemple sous forme de stick. Elle peut être utilisée comme produit de soin et/ou comme produit de maquillage de la peau, ou comme produit capillaire, par exemple comme shampooing ou après-shampooing.

De façon connue, la composition de l'invention peut contenir également les adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse, dans la phase aqueuse, dans les vésicules lipidiques et/ou dans les nanoparticules. Ces adjuvants, ainsi que leurs concentrations, doivent être tels qu'ils ne nuisent pas aux propriétés avantageuses du composé bicyclique aromatique selon l'invention.

Lorsque la composition selon l'invention est une émulsion, la proportion de la phase grasse peut aller de 5 à 80 % en poids, et de préférence de 5 à 50 % en poids par rapport au poids total de la composition. Les matières grasses, les émulsionnants et les coémulsionnants utilisés dans la composition sous forme d'émulsion sont choisis parmi ceux classiquement utilisés dans le domaine considéré. L'émulsionnant et le coémulsionnant sont de préférence présents, dans la composition, en une proportion allant de 0,3 à 30 % en poids, et de préférence de 0,5 à 20 % en poids par rapport au poids total de la composition.

Comme matières grasses utilisables dans l'invention, on peut utiliser, outre les solubilisants à base de 2-alkyl alcan-1-ols et de leurs esters, les huiles et notamment les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras tels que l'alcool cétylique, des acides gras, des cires et des gommes et en particulier les gommes de silicone.

Comme émulsionnants et coémulsionnants utilisables dans l'invention, on peut citer par exemple les esters d'acide gras et de polyéthylène glycol tels que le stéarate de PEG-100, le stéarate de PEG-50 et le stéarate de PEG-40 ; les esters d'acide gras et de polyol tels que le stéarate de glycéryle, le tristéarate de sorbitane et les stéarates de sorbitane oxyéthylénés disponibles sous les dénominations commerciales Tween® 20 ou Tween® 60, par exemple ; et leurs mélanges.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras et la silice hydrophobe.
Comme actifs, on peut utiliser notamment les dépigmentants, les émollients, les hydratants, les anti-séborrhéiques, les anti-acnéiques, les agents favorisant la repousse des cheveux, les agents kératolytiques et/ou desquamants, les agents apaisants et leurs mélanges. Avantageusement, dans la composition selon l'invention, les composés bicycliques aromatiques définis plus haut seront employés en combinaison avec d'autres composés à activité de type rétinoïde, avec des anti-radicaux libres, ou avec des α-hydroxy ou α-céto acides ou leurs dérivés. Par anti-radicaux libres, on entend par exemple l'α-tocophérol, la Super Oxyde Dismutate, l'Ubiquinol ou certains chélatants de métaux. Par α-hydroxy ou α-céto acides ou leurs dérivés, on entend par exemple les acides lactique, malique, citrique, glycolique, mandélique, tartrique, glycérique ou leurs sels, amides ou esters.

En cas d'incompatibilité, les actifs indiqués ci-dessus peuvent être incorporés dans des sphérules, notamment des vésicules ioniques ou non-ioniques et/ou des nanoparticules (nanocapsules et/ou nanosphères), de manière à les isoler les uns des autres dans la composition.

La composition selon l'invention trouve en particulier une application dans l'hygiène corporelle et capillaire et notamment pour le soin des peaux à tendance acnéique ou des peaux sèches, pour lutter contre l'aspect gras de la peau ou des cheveux, pour lutter contre la chute des cheveux, dans la protection contre les aspects néfastes du soleil ou pour prévenir et/ou pour lutter contre le vieillissement actinique ou chronologique.

La présente invention concerne donc également l'utilisation de la composition mentionnée ci-dessus pour le traitement cosmétique de la peau, en particulier contre l'acné et/ou le vieillissement chronologique ou actinique. Elle concerne aussi l'utilisation de cette composition pour le traitement cosmétique du cuir chevelu, en particulier pour lutter contre l'aspect gras des cheveux et la chute des cheveux.

La présente invention concerne également l'utilisation de la composition mentionnée ci-dessus pour fabriquer une préparation destinée à lutter contre l'acné et/ou le vieillissement chronologique ou actinique de la peau. Elle concerne aussi l'utilisation de la composition mentionnée ci-dessus pour fabriquer une préparation destinée à lutter contre l'aspect gras des cheveux et la chute des cheveux.

Dans tous les cas, la composition selon l'invention et/ou la préparation obtenue à partir de celle-ci comprend une quantité efficace de composé bicyclique aromatique, suffisante pour obtenir l'effet recherché, et un milieu physiologiquement acceptable L'invention sera maintenant illustrée par les exemples non limitatifs suivants. Dans ces exemples, les quantités sont indiquées en pourcentage pondéral, sauf indication contraire.

### EXEMPLES

On prépare six compositions C1 à C5 telles que définies dans le Tableau 1.

Dès sa formation, la composition C1 comprend des cristaux visibles au microscope. Dans la composition C2, le composé A est mieux solubilisé, mais quelques cristaux sont toutefois visibles après un mois à température ambiante. Les compositions C3 à C5 ne présentent pas de cristaux après deux mois à température ambiante, la composition C5 n'en comprenant pas non plus à 4°C, 37°C et 45°C après cette période de temps.

Il ressort donc clairement du Tableau 1 que le 2-hexyl-1-décanol permet de solubiliser le composé A, en particulier lorsque le rapport pondéral de l'alcool de Guerbet au composé A est d'au moins 20:1, de préférence d'au moins 30:1.

L'invention n'est toutefois pas limitée à ce solubilisant particulier et s'étend à tout alcool de Guerbet et ester d'un tel alcool.

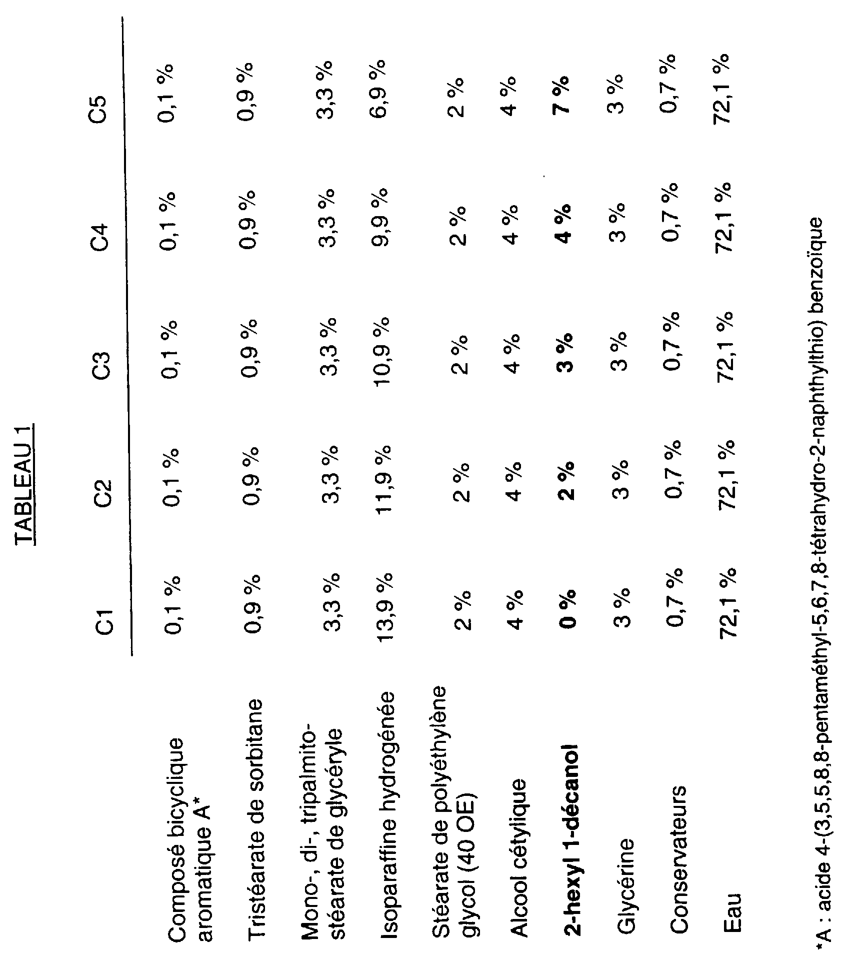

## Revendications

1. Composition comprenant au moins un composé bicyclique aromatique ayant la formule générale (I) suivante : 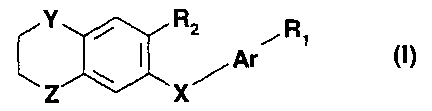 dans laquelle :
* R₁ représente un atome d'hydrogène, un radical -CH₃, un radical -CH₂-O-R₃, un radical -CH₂-O-CO-R₄, un radical -O-R₅, un radical -O-(CH₂)ₘ-(CO)ₙ-R₆, un radical -CO-R₇, un radical -CO-O-R₈, ou encore un radical -S(O)ₚ-R₉, les valeurs m, n et p ainsi que les différents radicaux R₃ à R₉ ayant la signification donnée ci-après,
* R₂ représente un atome d'hydrogène ou un atome d'halogène, un radical alkyle inférieur, un radical -NO₂, un radical -O-COR₄, un radical -OR₉ ou bien encore un radical 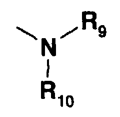 les radicaux R₄, R₉, et R₁₀ ayant la signification donnée ci-après,
* Ar représente un radical choisi parmi les radicaux de formules (a)-(e) suivantes : 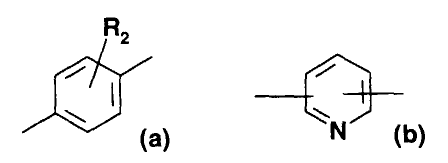 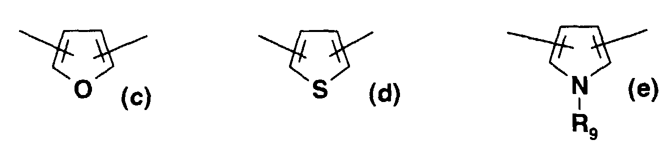 dans lesquelles R₂ a la signification ci-dessus, et R₉ celle donnée ci-après,
* X représente -O-, -S(O)ₜ- ou un radical -NR₉-, la valeur t et le radical R₉ ayant la signification donnée ci-après,
* Y et Z représentent -O-, -S(O)ₜ- ou encore un radical -CR₁₁R₁₂, la valeur t et les radicaux R₁₁ et R₁₂ ayant la signification donnée ci-après,
étant entendu que dans tout ce qui précède :
- m est un nombre entier égal à 1, 2 ou 3, n est un nombre entier égal à 0 ou 1, p est un nombre entier égal à 0, 1, 2 ou 3 et t est un nombre entier égal à 0, 1 ou 2,
- R_{3,} R_{5,} R_{9,} R_{10,} R_{11,} R₁₂ représentent, indépendamment les uns des autres, un atome d'hydrogène ou un radical alkyle inférieur,
- R₄ représente un radical alkyle inférieur,
- R₆ représente un radical alkyle inférieur ou un hétérocycle,
- R₇ représente un atome d'hydrogène, un radical alkyle inférieur ou un radical : 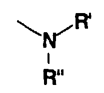 dans lequel R' et R", identiques ou différents, représentent un atome d'hydrogène, un radical alkyle inférieur, un radical mono ou polyhydroxyalkyle, un radical aryle éventuellement substitué ou un reste d'aminoacide ou de peptide ou de sucre ou encore, pris ensemble, forment un hétérocycle,
- R₈ représente un atome d'hydrogène, un radical alkyle linéaire ou ramifié ayant de 1 à 20 atomes de carbone, un radical alkényle, un radical mono ou polyhydroxyalkyle, un radical aryle ou aralkyle éventuellement substitué ou un reste de sucre ou un reste d'aminoacide ou de peptide,
- Y et Z ne peuvent représenter en même temps un atome d'oxygène ou un radical -S(O)ₜ-.
ou un sel ou analogue chiral d'un tel composé, caractérisée en ce qu'elle comprend en outre au moins un 2-alkyl alcan-1-ol comprenant de 12 à 36 atomes de carbone, ou un ester d'un tel alcool.

2. Composition selon la revendication 1, caractérisée en ce que ledit composé de formule (I) est tel que l'une au moins, et de préférence toutes, les conditions ci-dessous sont respectées :
- R₁ est un radical -CO-R₇
- R₂ est un radical alkyl inférieur ou un radical -OR₉
- Ar représente un radical de formule (a)
- et X représente -O-, -S- ou -NR₉-.

3. Composition selon la revendication 1 ou 2, caractérisée en ce que ledit composé de formule (I) est choisi parmi :
- l'acide 4-(5,6,7,8-tétrahydro-5,5,8,8-tétraméthyl-2-naphthyloxy) benzoïque
- l'acide 4-(5,6,7,8-tétrahydro-5,5,8,8-tétraméthyl-2-naphthylthio) benzoïque
- l'acide 4-(5,6,7,8-tétrahydro-5,5,8,8-tétraméthyl-2 naphthylsulfinyl) benzoïque
- l'acide 4-(5,6,7,8-tétrahydro-5,5,8,8-tétraméthyl-2-naphthylsulfonyl) benzoïque
- l'acide 4-(5,6,7,8-tétrahydro-5,5,8,8-tétraméthyl-2-naphthylamino) benzoïque
- l'acide 5-(5,6,7,8-tétrahydro-5,5,8,8-tétraméthyl-2-naphthylthio)-2-thiophène carboxylique
- l'acide 4-(3,5,5,8,8-pentaméthyl-5,6,7,8-tétrahydro-2-naphthyloxy) benzoïque
- l'acide 4-(3,5,5,8,8-pentaméthyl-5,6,7,8-tétrahydro-2-naphthylthio) benzoïque
- l'acide 4-(3-éthyl-5,5,8,8-tétramethyl-5,6,7,8-tétrahydro-2-naphthyloxy) benzoïque
- l'acide 4-(3-isopropyl-5,5,8,8-tétramethyl-5,6,7,8-tétrahydro-2-naphthyloxy) benzoïque
- la 4-(5,6,7,8-tétrahydro-5,5,8,8-tétraméthyl-2-naphthyloxy) acétophénone
- le 4-(5,6,7,8-tétrahydro-5,5,8,8-tétraméthyl-2-naphthyloxy) benzaldéhyde
- l'acide 4-(3-bromo-5,6,7,8-tétrahydro-5,5,8,8-tétramethyl-2-naphtyloxy) benzoïque
- l'acide 3-méthyl-4-(5,6,7,8-tétrahydro-5,5,8,8-tétramethyl-2-naphtylthio) benzoïque
- l'acide 3-méthyl-4-(3,5,5,8,8-pentaméthyl-5,6,7,8-tétrahydro-2-naphtylthio) benzoïque
- l'acide 3-méthyl-4-(3-éthyl-5,6,7,8-tétrahydro-5,5,8,8-tétramethyl-2-naphtyloxy) benzoïque
- l'acide 6-(3,5,5,8,8-pentaméthyl-5,6,7,8-tétrahydro-2-naphtylthio) nicotinique
- l'acide 2-hydroxy-4-(3,5,5,8,8-pentaméthyl-5,6,7,8-tétrahydrol-2-naphtylthio) benzoïque
- l'acide 2-chloro-4-(3,5,5,8,8-pentaméthyl-5,6,7,8-tétrahydro-2-naphtylthio) benzoïque
- l'acide 4-(3-éthyl-5,6,7,8-tétrahydro-5,5,8,8-tétramethyl-2-naphtylthio) benzoïque
- l'acide 4-(3-isopropyl-5,6,7,8-tétrahydro-5,5,8,8-tétramethyl-2-naphtylthio) benzoïque
- l'acide 4-(3-n-propyl-5,6,7,8-tétrahydro-5,5,8,8-tétramethyl-2-naphtylthio) benzoïque
- le 4-(3,5,5,8,8-pentaméthyl-5,6,7,8-tétrahydro-2-naphtylthio) benzèneméthanol
- le 4-(3,5,5,8,8-pentaméthyl-5,6,7,8-tétrahydro-2-naphtylthio) benzaldéhyde
- la N-éthyl-4-(3,5,5,8,8-pentaméthyl-5,6,7,8-tétrahydro-2-naphtylthio) benzamide
- la N-4-hydroxyphényl-4-(3,5,5,8,8-pentaméthyl-5,6,7,8-tétrahydro-2-naphtylthio) benzamide.

4. Composition selon la revendication 3, caractérisée en ce que ledit composé de formule (I) est l'acide 4-(3,5,5,8,8-pentaméthyl-5,6,7,8-tétrahydro-2-naphthylthio) benzoïque.

5. Composition selon l'une quelconque des revendications précédentes, caractérisée en ce qu'elle comprend de 0,0001% à 10% en poids de composé de formule (I).

6. Composition selon la revendication 5, caractérisée en ce qu'elle comprend de 0,01 à 5% en poids de composé de formule (I).

7. Composition selon la revendication 6, caractérisée en ce qu'elle comprend de 0,1 à 1% en poids de composé de formule (I).

8. Composition selon l'une quelconque des revendications précédentes, caractérisée en ce que ledit 2-alkyl alcan-1-ol comprend de 16 à 20 atomes de carbone.

9. Composition selon l'une quelconque des revendications précédentes, caractérisée en ce que ledit 2-alkyl alcan-1-ol est choisi parmi : le butyloctanol, l'hexyldécanol, l'octyldécanol, l'alcool isostéarylique, l'octyldodécanol, le décyltétradécanol, le undécylpentadécanol, le dodécylhexadécanol, le tétradécyloctadécanol, l'hexyldécyloctadécanol, le tétradécyleicosanol, le cétylarachidol et le mélange d'alcools iso-cétylique, iso-stéarylique et iso-arachidylique.

10. Composition selon la revendication 6, caractérisée en ce que ledit 2-alkyl alcan-1-ol est le 2-hexyl décanol.

11. Composition selon l'une quelconque des revendications précédentes, caractérisée en ce qu'elle comprend une quantité pondérale de 2-alkyl alcan-1-ol ou de son ester qui est au moins vingt fois supérieure à la quantité pondérale de composé bicyclique aromatique.

12. Composition selon la revendication 9, caractérisée en ce qu'elle comprend une quantité pondérale de 2-alkyl alcan-1-ol ou de son ester qui est au moins trente fois supérieure à la quantité pondérale de composé bicyclique aromatique.

13. Composition selon l'une quelconque des revendications précédentes, caractérisée en ce qu'elle comprend de 0,01% à 20% en poids de 2-alkyl alcan-1-ol.

14. Composition selon l'une quelconque des revendications précédentes, caractérisée en ce qu'elle comprend de 1% à 10% en poids de 2-alkyl alcan-1-ol.

15. Utilisation de la composition selon l'une quelconque des revendications précédentes pour le traitement cosmétique de la peau, en particulier contre l'acné et/ou le vieillissement chronologique ou actinique.

16. Utilisation de la composition selon l'une quelconque des revendications 1 à 14 pour le traitement cosmétique du cuir chevelu, en particulier pour lutter contre l'aspect gras des cheveux et la chute des cheveux.

17. Utilisation de la composition selon l'une quelconque des revendications 1 à 14 pour fabriquer une préparation destinée à lutter contre l'acné et/ou le vieillissement chronologique ou actinique de la peau.

18. Utilisation de la composition selon l'une quelconque des revendications 1 à 14 pour fabriquer une préparation destinée à lutter contre l'aspect gras des cheveux et la chute des cheveux.

19. Procédé de solubilisation composé bicyclique aromatique ayant la formule générale (I) suivante : 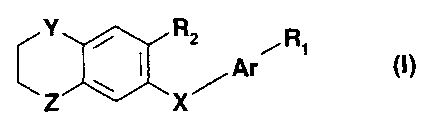 dans laquelle :
* R₁ représente un atome d'hydrogène, un radical -CH₃, un radical -CH₂-O-R₃, un radical -CH₂-O-CO-R₄, un radical -O-R₅, un radical -O-(CH₂)ₘ-(CO)ₙ-R₆, un radical -CO-R₇, un radical -CO-O-R₈, ou encore un radical -S(O)ₚ-R₉, les valeurs m, n et p ainsi que les différents radicaux R₃ à R₉ ayant la signification donnée ci-après,
* R₂ représente un atome d'hydrogène ou un atome d'halogène, un radical alkyle inférieur, un radical -NO₂, un radical -O-COR₄, un radical -OR₉ ou bien encore un radical 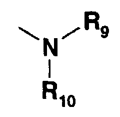 les radicaux R₄, R₉, et R₁₀ ayant la signification donnée ci-après,
* Ar représente un radical choisi parmi les radicaux de formules (a)-(e) suivantes : 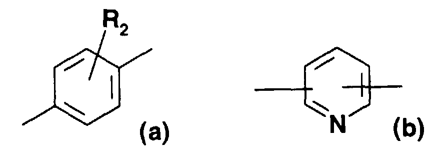 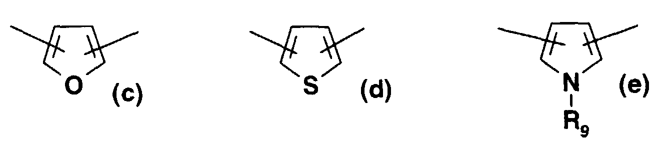 dans lesquelles R₂ a la signification ci-dessus, et R₉ celle donnée ci-après,
* X représente -O-, -S(O)ₜ- ou un radical -NR₉-, la valeur t et le radical R₉ ayant la signification donnée ci-après,
* Y et Z représentent -O-, -S(O)ₜ- ou encore un radical -CR₁₁R₁₂, la valeur t et les radicaux R₁₁ et R₁₂ ayant la signification donnée ci-après,
étant entendu que dans tout ce qui précède :
- m est un nombre entier égal à 1, 2 ou 3, n est un nombre entier égal à 0 ou 1, p est un nombre entier égal à 0, 1, 2 ou 3 et t est un nombre entier égal à 0, 1 ou 2,
- R_{3,} R_{5,} R_{9,} R_{10,} R_{11,} R₁₂ représentent, indépendamment les uns des autres, un atome d'hydrogène ou un radical alkyle inférieur,
- R₄ représente un radical alkyle inférieur,
- R₆ représente un radical alkyle inférieur ou un hétérocycle,
- R₇ représente un atome d'hydrogène, un radical alkyle inférieur ou un radical : 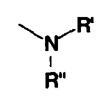 dans lequel R' et R", identiques ou différents, représentent un atome d'hydrogène, un radical alkyle inférieur, un radical mono ou polyhydroxyalkyle, un radical aryle éventuellement substitué ou un reste d'aminoacide ou de peptide ou de sucre ou encore, pris ensemble, forment un hétérocycle,
- R₈ représente un atome d'hydrogène, un radical alkyle linéaire ou ramifié ayant de 1 à 20 atomes de carbone, un radical alkényle, un radical mono ou polyhydroxyalkyle, un radical aryle ou aralkyle éventuellement substitué ou un reste de sucre ou un reste d'aminoacide ou de peptide,
- Y et Z ne peuvent représenter en même temps un atome d'oxygène ou un radical -S(O)ₜ-.
ou un sel ou analogue chiral d'un tel composé, caractérisé en ce que ledit procédé comprend l'étape consistant à mélanger ledit composé bicyclique à au moins un 2-alkyl alcan-1-ol comprenant de 12 à 36 atomes de carbone, ou à un ester d'un tel alcool.
